# EUROPEAN PATENT APPLICATION

(11) **EP 2 644 092 A1**
(43) Date of publication of application: **02.10.2013**
(21) Application number: 13161948.8
(22) Date of filing: 02.04.2013
(51) Int. Cl.: A61B 5/055, G01R 33/20, G01R 33/48, A61B 5/00

(54) **Method for localisation and/or quantification of 19F-containing compounds via 19F magnetic resonance (MR) analysis after topical administration**

(30) Priority: 30.03.2012 EP 12162476; 12.06.2012 EP 12171681
(71) Applicant: Max-Delbrück-Centrum für Molekulare Medizin, 13125 Berlin (DE)
(72) Inventor: Niendorf, Thoralf, 52074 Aachen (DE); Waiczies, Helmar, 13353 Berlin (DE); Waiczies, Sonia, 13353 Berlin (DE)
(74) Representative: Lange, Sven

(57) **Abstract**

The invention relates to analytical approaches towards determining drug bioavailability and the development of topical formulations for pharmaceutical products. The invention relates to a non-invasive method for localisation and/or quantification of ¹⁹F-containing compounds via ¹⁹F magnetic resonance (MR) imaging or spectroscopy, after topical administration of said compound to a subject. The invention also relates to an MR-system for conducting said method.

## Description

The invention relates to the field of analytical methods for determining drug bioavailability after topical administration and the development of topical formulations for pharmaceutical products. The invention relates to a non-invasive method for localisation and/or quantification of ¹⁹F-containing compounds via ¹⁹F magnetic resonance (MR) imaging or spectroscopy after topical administration of said compound to a subject. The invention also relates to an MR-system for conducting said method, based preferably - but not exclusively - on a double-tuned ¹⁹F/¹H radio frequency probe using a birdcage resonator as an example.

### BACKGROUND OF THE INVENTION

The topical bioavailability of a pharmacological compound is defined as the rate and extent at which a drug reaches its site of action following topical application. It is often difficult to calculate the topical bioavailability over time for pharmacological compounds that act at sites deeper than their application site. It is also usually uncertain - for most pharmacological compounds - which concentration of active compound is required locally to exert a pharmacological effect.

The bioavailability of systemically-delivered therapeutic compounds is typically defined as the rate and extent at which a drug reaches the general circulation from an administered dosage form. For such compounds measurement of drug levels in the blood gives a good indication of drug bioavailability following administration. However, the systemic availability of topically applied drug preparations - that are designed to exert localized effects - is minimal. Therefore blood measurements are not suitable for measuring drug bioavailability of topically applied drugs (e.g. in the form of creams). Even in the case of systemically applied drugs, the pharmacodynamics are influenced by enzymatic interactions and transport mechanisms; thus it may be assumed that blood concentration measurements of the drug do not provide an accurate picture of the drug availability at the target tissue or organ system. Indeed continuous sampling of active drug in the tissues is the most rational approach to estimate active drug profiles at the site of action.

Especially for topically applied drugs, a valid method that quantifies the skin permeation and bioavailability of active compound in the skin and surrounding tissue following topical application is imperative. Methods currently employed for the measurement of topical bioavailability include (i) dermatopharmacokinetic (DPK) analysis (also skin or tape stripping), (ii) dermal microdialysis and (iv) Raman and near infrared (NIR) spectroscopy.

The first method is a minimally-invasive procedure that involves sequential removal of microscopic layers of stratum corneum (SC), the outermost layer of the skin. One major drawback of the DPK method is that drug concentration can only be quantified at this level; for tissue levels lower than this, the drug concentration can only be extrapolated, assuming a linear correlation in drug availability during percutaneous absorption [1]. Although this method has long been in use it is still not completely optimized. A draft guidance issued by the FDA on this method was withdrawn soon after contradictory results by an advisory committee were reported on the FDA web site. Notwithstanding these reports, improved designs for the DPK method are ongoing; in particular considerations are made regarding duration of contact of the pharmacological compound with the application site and removal of excess product from the skin, control of environmental conditions (mainly temperature and humidity) during the study and optimization of quantification (including normalization of the drug penetration data with SC thickness that can vary from 5 to 20 pm in the forearm) [2].

The second method, microdialysis (MD), is also a minimally-invasive procedure that involves placing an ultrathin probe consisting of a hollow fiber in the dermis. A microdialysis pump allows the slow perfusion of sterile buffer through the probe catheter [3]. The semi-permeability of the tube allows for exchange of small molecules between the extracellular space and the probe. Apart from concentration of active compound it is also possible to measure biological markers produced as a result to a response to the active compound with this method. Similar to oral absorption experiments, the MD method provides concentration-time profiles that allow pharmacokinetic and bioavailability measurements [4]. MD is a newer and relatively less explored technique compared to DPK analysis. Drawbacks for this technique include invasiveness (causing tissue trauma and inflammation), the necessary skill and proper training to insert probe at a consistent depth in the skin, a low recovery for lipophilic and highly protein-bound drugs and large variability in the measurements and the limited depth penetration.

The third method for measuring the topical bioavailability of pharmacological compounds is spectroscopy. Raman spectroscopy (RS) has been commonly employed; it is a semi quantitative technique that identifies molecular structures with the aid of a confocal laser that penetrates the skin. Following interaction with compounds within the skin, photons are scattered back, captured and analyzed according to their specific Raman spectra. The method can be utilized to measure a number of compounds such as urea [5] due to their unique Raman signature which allows them to be differentiated from other constituents of the skin. Although an ideal technique regarding patient comfort (not invasive), this method still suffers a number of limitations. The molecule of interest should possess a distinct spectrum with a sufficient intensity in order to be differentiated from other skin constituents, thus limiting its universal use. Furthermore this spectroscopic technique is limited to measurements across the SC; measurements are commonly recorded from the skin surface down to a depth of 24 pm, in 2 pm steps. Near infra-red (NIR) spectroscopy and its absorbance technology allows deeper measurements and be used to quantify the diffusion rate of a pharmacological compound through the skin by using linear multivariate statistics [6]. The NIR method appears to be sensitive and its results correlate with those from HPLC analysis following tissue extraction [6]. Currently it appears to be the best of the available methodologies since it is quantitative, rapid and nondestructive; however its application for topical bioavailability measurements is still in its exploratory phase. Furthermore, the lasers from the NIR technology can penetrate the skin deeper but are associated with a signal weakness in depth. Although this can be improved with time-domain methods (Fourier transform IR, FTIR) these are associated with increased acquisition time. Raman and NIR spectroscopy share a very limited depth penetration in common.

While NIR spectroscopy appears to be the most promising of the available technologies to determine the bioavailability of topically applied pharmacological compounds, none of the available techniques provide sufficient depth detail for compounds whose actions lie deeper than the adipose tissue. Furthermore none of the available technologies provide anatomical detail that would specify the distribution of the compound, especially in relation to the pathology. Both the localization and quantification of an active compound following topical application are valuable sets of information that are necessary to determine the quantity and proximity of the pharmacological substance to the area of pathology and thus site of action.

As mentioned above, a number of methods are available to quantify the skin permeation and bioavailability of active compound in the skin following topical application. These methods include DPK analysis, dermal microdialysis and Raman/ NIR spectroscopy. Although these methods have proved to be promising for determining the level of skin permeation and bioavailability of pharmacological compounds in the skin, their main drawback is the depth limitation, low resolution and thus poor quantification of active compound in deeper tissue.

The currently available methods for measuring the local bioavailability of pharmacological compounds are capable of performing measurements at a limited depth beneath the skin surface. Even for the more novel and explorative methods involving NIR technology the maximum depth that can be reached is limited to a few millimeters beneath the skin surface and the signal sensitivity decreases with depth. These methods are therefore not applicable for pharmacological compounds that are applied topically and are meant to act at sites that are deeper than a few millimeters beneath the skin surface. For example Non-Steroidal Anti-Inflammatory Drug (NSAIDs) have been shown to have anti-inflammatory, analgesic, and antipyretic effects and when applied topically they are believed to reduce inflammation within muscles and joints. These compounds have been shown to penetrate the human skin but there is no methods described that can quantify the levels of active compound at the site of injury where the compound is acting.

The analysis of ¹⁹F-containing compounds as such, for example perfluorinated compounds, using ¹⁹F MRI is known in the art. WO 2011/103138 discloses for example the use of MRI to measure perfluorinated gas in the lung of a subject in order to investigate pulmonary lung function. Measurement of a substance having passed through the surface of the lung is not disclosed. Flögel et al at the 19th Annual Meeting of the ISMRM disclose the use of emulsified perfluorocarbons (PFCs), which are phagocytised by monocytes/macrophages and therefore readily detected by ¹⁹F MRI. This analysis focused on the detection of inflammation itself via detection of the cells labelled with PFCs. Celsense have also disclosed a similar approach based on the uptake of perfluorocarbon particles in leukocytes, followed by ¹⁹F NMR/MRI. ¹⁹F-containing compounds are as such known in the prior art in the field of medicine [8]. Until the present time ¹⁹F MRI has however never been applied to determine topically applied compounds in order to localise and/or quantify the distribution of said administered compound. In the field of drug bioavailability there is a significant lack of suitable quantitative means for investigating localisation or distribution of topically applied substances.

### SUMMARY OF THE INVENTION

In light of the prior art, the technical problem underlying the present invention is to provide means for determining the location, quantity and/or local bioavailability of pharmacological compounds that are applied topically.

This problem is solved by the features of the independent claims. Preferred embodiments of the present invention are provided by the dependent claims.

Therefore, an object of the invention is to provide a non-invasive method for localisation and/or quantification of ¹⁹F-containing xenobiotic compounds via ¹⁹F magnetic resonance (MR) analysis after topical administration of said compound to a subject.

In a preferred embodiment the method is **characterised in that** the MR analysis is ¹⁹F/¹H MR. Through a combination of ¹⁹F MR and other MR detectable nuclei (for example ¹H, 23Na, 31P, 13C etc.) the physiology of the subject may be analysed and subsequently compared to the ¹⁹F MR signal, thereby providing useful information on localisation/distribution of the administered compound together with the anatomy, morphology, function, electrolyte content and energy metabolism.

A preferred embodiment of the invention therefore relates to the method as described herein, whereby proton ¹H anatomical images and ¹⁹F images are combined and/or compared, for example by overlay. The method of the present invention provides surprisingly clear images at high resolution that enable precise localisation of the administered compound at depths in tissue well below the point of topical administration. The present method therefore represents significant technical progress in the field of determining the local bioavailability of pharmacological compounds that are applied topically.

In one embodiment of the invention the method as described herein is **characterised in that** the MR analysis is - but is not restricted to - T1- and/or T2- or diffusion weighted MR measurements.

In one embodiment of the invention the method as described herein is **characterised in that** the MR analysis is carried out with - but not restricted to - a 7 T MR device. In a preferred embodiment the MR analysis is carried out with an MR device of 7 T or above, preferably 7 T or 14 T. Higher field strengths correlate with higher signal to noise ratio (SNR) and therefore higher sensitivity. The invention therefore relates to the use of MR devices such as MRI of 7 T, more than 7 T, more than 8 T, more than 9 T, more than 10 T, more than 11 T, more than 12 T, more than 13 T or 14 T or more than 14 T. A 9.4 T device may also be used in the method of the present invention.

Until the present time, the use of MR devices with higher field strengths has not been disclosed in the art. The combination of higher field strengths (for example 7 T or above) with an analysis of topically administered compounds provides unexpected advantages in providing a solution to the problem of the invention. The higher resolution enabled by the analysis at higher field strengths provides a resolution and signal to noise ratio that is particularly relevant for topically administered compound, as the resolution is sufficient to provide information on the location and distribution of compound within the various layers of the epithelium, for the example the skin. Differentiation between epidermis, dermis, hypodermis and musculoskeletal localisation provides significant information on the effectiveness of any given formulation for topical administration.

In one embodiment of the invention the method as described herein is **characterised in that** the ¹⁹F-containing xenobiotic compound is Glucocorticosteroid (GC) or Non-Steroidal Anti-Inflammatory Drug (NSAID). These two categories of compound are commonly administered via topical application and are subject to structural modification, or to modification in their clinical formulation, in order to improve their biological distribution properties. Significant efforts are made to enable topical application of such compounds in order to treat immunologically-related or inflammation-related diseases more effectively, for example Rheumatoid arthritis.

In one embodiment of the invention the method as described herein is **characterised in that** subject is an animal, preferably a mammal, more preferably a human.

In one embodiment of the invention the method as described herein is **characterised in that** local concentration and/or bioavailability of a pharmacological compound in body tissue is determined irrelevant of depth from the administration site of said compound. One of the surprising and advantageous aspects of the present invention in comparison to those methods known in the art for determining the in vivo distribution of topically applied compounds is that measurements can be conducted well below the surface of the administration site. In a preferred embodiment the skin or mucosal tissue (lung, nasal, mucosa, vaginal etc.) of a subject is treated with any given ¹⁹F-containing compound. According to the methods of the prior art, information regarding the biological distribution (availability) of the compound would not have been easily obtained due to the limitations of the older techniques as described above. The ability to measure (locate/quantify) the administered compound, even centimetres below the treated area of application, represents a significant advance in the analysis of topically administered biological compounds.

The method of the present invention is highly relevant, if not essential, for the development and legal approval of novel ¹⁹F containing drugs, including the over-the-counter mass market of anti-inflammatory topical crèmes. Not to mention the ever growing market of anti-aging crèmes and Spa merchandises. In general, localization and quantification of an active compound following topical application are valuable sets of information that are necessary to determine the quantity and proximity of the pharmacological substance to the area of pathology and thus site of action.

In one embodiment of the invention the method as described herein is **characterised in that** the location of disease- or pathologically-related physiological features, such as areas of inflammation, infection, or other pathologically relevant physiological feature, is additionally determined using ¹H MR. ¹H MR enables a detailed and reliable imaging of the tissue of the subject to be analysed. Through the ¹H MR analysis disease- or pathologically-related physiological features, such as areas of inflammation, infection, or other pathologically relevant physiological features may be determined and a measurement of the bioavailability of the administered compound provided.

The present invention therefore also relates to the use of the method as described herein for the determination of bioavailability of a ¹⁹F-containing xenobiotic compound after topical administration of said compound to a subject.

The invention also relates to the use of the method as described herein for optimising the bioavailability of a topically applied ¹⁹F-containing xenobiotic compound by comparing the in vivo distribution of said compound after administration in multiple pharmaceutically acceptable formulations followed by subsequent modification of said formulations. Multiple samples, each containing different topical formulations for any given substance could be tested and the effectiveness (for example the depth of penetration) of the administered compound could be tested and compared. The analysis of the present invention provides, in the context of bioavailability of topically administered active agents, an enormous advance in technology. The method allows systematic quantitative analysis of novel topical formulations of active compounds, thereby enabling the production of improved topical medicaments at a rate and reliability that was until now not possible.

A further aspect of the invention relates to a system for the localisation and/or quantification of ¹⁹F-containing compounds comprising single (¹⁹F) or multi-tunable (for example ¹⁹F/¹H, ¹⁹F/¹H/²³Na, ¹⁹F/¹H/³¹P) RF coils and an MR apparatus. The system has been specifically designed for carrying out the method of the present invention and enables the analysis as described herein.

One embodiment of the invention therefore relates to a system for the localisation and/or quantification of ¹⁹F-containing compounds, whereby according to the anatomy of the region of interest different radio frequency probes (sometime referred to as coils) are applied (eg. birdcage designs for - but not exclusively for - imaging appendages such as limbs, surface coils for localized body surfaces and/or an integration of both to accommodate for different shapes and structures) in order to detect and acquire the different signals and to quantify the bioavailability of ¹⁹F-containing compounds.

In a preferred embodiment the system comprises a double-tuned ¹⁹F/¹H birdcage resonator designed for hand and wrist anatomy. The physical dimension of the coil should accommodate for and fit as closely as possible to the shape of the anatomical area of interest and should provide a reasonable close proximity to the imaging site without exceeding limits related to radio frequency (RF) radiation, namely the SAR (specific absorption rate), determined by the international electrotechnical commission (IEC).

The present invention is particularly advantageous due to the high resolution of the MR analysis, which enables determination and quantification of the distribution of any given ¹⁹F-containing compound within but not restricted to the herein-mentioned layers of skin. The high submillimetric spatial resolution and high signal to noise ratio (SNR), even in areas several centimeters below the surface of application, allow determination of whether any given compound administered topically has passed beyond each of the deeper layers described herein. This may relate to particularly significant information in the context of developing novel formulations for topical administration of pharmaceutical agents. At the present time the means for determining penetration of pharmaceutical agents into and in some cases through the skin (eg. topical application for treating musculoskeletal ailments) are substantially inadequate due to lack of resolution and particularly lack of accurate measurement at lower depths of the skin or even within musculoskeletal structures. The determination of the distribution of the topically applied compound within the skin (but not exclusively to this topical region) is one important aspect of the present invention and represents a significant improvement on those methods known in the art. In general, the higher the spatial resolution provided by the analysis technique, the better the analysis will be. With a 7 T MR device a resolution of 2mm x 2mm x 5mm can be achieved, with a 14 T MR device we can achieve a resolution of 1 mm x 1 mm x 2.5mm. These improvements in resolution are expected to improve even further by optimizing the performance of the radio frequency probes outlined herein.

In light of the prior art in the field of determining drug bioavailability and the development of topical formulations for pharmaceutical products, a skilled person would not have considered applying ¹⁹F MR. No suggestion has been made previously in the art that ¹⁹F MR could provide a solution for localisation of topically applied compounds with the resolution and reproducibility as demonstrated by the present inventors. The determination of ¹⁹F containing drugs in the patient's plasma has been typically measured with NMR (nuclear magnetic resonance) methods using patient plasma and not the patient him/herself. Previous approaches towards this kind of analysis have been plagued by insufficient sensitivity and signal intensity, which are two considerable issues that have not encouraged this method to be implemented earlier. In a preferred embodiment of the invention the increasing higher field strengths, such as disclosed herein, enable the implementation of the method of the present invention. The higher field strengths allow high resolution and accuracy, which was previously unable in those methods of the art with regard to measuring topically applied xenobiotic substances.

### DETAILED DESCRIPTION OF THE INVENTION

The invention concerns the use of an MR apparatus for a non-invasive localization and quantification of ¹⁹F-containing xenobiotic compounds and a system for localization and quantification of ¹⁹F-containing compounds comprising single (¹⁹F) or multi-tunable (for example ¹⁹F/¹H, ¹⁹F/¹H/²³ Na, ¹⁹F/¹H/³¹P) RF coils and an MR apparatus.

This invention preferably relates to magnetic resonance imaging (MRI), also referred to as nuclear magnetic resonance (NMR) imaging but also covers magnetic resonance spectroscopy (MRS). The techniques of MRI or NMR encompass the detection of atomic nuclei with magnetic fields and radio frequency (RF) radiation. The term MRI as used herein may therefore relate to either imaging (MRI) or spectroscopy (MRS), whereby the terms may be used interchangeably. The term MR as used herein is therefore intended to encompass all magnetic resonance variants. A preferred embodiment relates to MRI.

The present invention employs ultra-high field MR to specifically detect fluorine (¹⁹F) nucleus-derived magnetic resonance signals from ¹⁹F-containing xenobiotic compounds in relation to proton (¹H) signals from the tissue environment. This approach enables determination of xenobiotic compounds in relation to the anatomy of the area of application (and also site of action) at high submillimetric spatial resolution and high signal to noise ratio (SNR) even in areas several centimeters below the skin surface. ¹⁹F atoms give a clear NMR signal and thus may function as suitable probe in MR. The specific advantages emerging from the use of ¹⁹F are: (i) a virtual absence of ¹⁹F in the body tissues that yields background free images with complete signal selectivity and (ii) a possibility to quantity the signal by ¹⁹F NMR spectroscopy.

This invention involves preferably the application of ¹⁹F MR to localize and quantify ¹⁹F-containing xenobiotic compounds, for example pharmacological agents that are administered topically (in the form of creams, ointments, lotions, milk, shampoos, drops, lozenges, sprays, inhalers etc.) to different parts of the human body (eg. skin, scalp, eyes, nose, mouth).

Examples of ¹⁹F-containing xenobiotics that are included in this invention are those from the Glucocorticosteroid (GC) group (eg. fluocortolone, diflorasone, fluocinonide, betamethasone) and Non-Steroidal Anti-Inflammatory Drug (NSAID) group (eg. flufenamic acid, niflumic acid). Due to their anti-inflammatory properties topically-applied GC and NSAID are commonly prescribed for the treatment of inflammation in topical surfaces, namely mucosal membranes (eg. ocular, nasal, buccal, oropharyngeal, bronchial, gastric, penile, uterine etc.) and skin.

NSAIDs have also been shown to have analgesic and antipyretic effects and when applied topically they are used to reduce local inflammation in various locations such as joints and muscles in the case of rheumatoid arthritis and (sport) trauma, oropharynx in the case of pharyngitis etc. Although it is known that drugs such as NSAIDs can penetrate the human skin [7], it is not clear what quantities of active compound reaches the site of pathology, where the compound is believed to act. This is particularly significant for pathologies (eg. rheumatoid arthritis) that are present in tissues up to several centimeters beneath the skin surface.

The invention also concerns a system for localization and quantification of ¹⁹F-containing compounds comprising single (¹⁹F) or multi-tunable (for example ¹⁹F/¹H, ¹⁹F/¹H/²³Na, ¹⁹F/¹H/³¹P) RF coils. The system achieves surprisingly the localization and quantification of ¹⁹F-containing compounds, including NSAIDs, in different parts of the body. For this, single (¹⁹F) or multi-tunable (for example ¹⁹F/¹H, ¹⁹F/¹H/²³Na, ¹⁹F/¹H/³¹P) radiofrequency antennae, so called RF coils are applied. According to the anatomy of the region of interest different RF coils are optimized to detect and acquire the different signals and to quantify the bioavailability of ¹⁹F-containing compounds. Furthermore, it is preferred that the system comprises for the application of ¹⁹F/¹H imaging and spectroscopy a double-tuned ¹⁹F/¹H RF probe, for example an double-tuned ¹⁹F/¹H RF birdcage resonator designed for hand and wrist anatomy (Figure la). The coil was built using the design derived from electromagnetic field (EMF) simulations. The use of the double- or multi-tuned RF coils shows surprising advantages in light of the present technological advancement in the field. The multi-tunable (for example ¹⁹F/¹H, ¹⁹F/¹H/²³ Na, ¹⁹F/¹H/³¹P) radiofrequency antennae provide a surprisingly fast and accurate determination of ¹⁹F-containing compounds. The method as described herein and the present system enable automated and rationalised approaches towards the development of pharmaceutical and/or cosmetic formulations intended for topical application until now not disclosed in the art.

¹⁹F MR methods are employed in the present invention involving both imaging and spectroscopy to make a quantitative assessment of ¹⁹F-containing xenobiotic compounds in vivo following topical application. This provides an alternate means to the above methodologies for measuring the in vivo bioavailability of pharmacological agents that contain one or more ¹⁹F atoms in their molecular structure. In addition, the present invention enables determination of the proximity of the ¹⁹F-containing xenobiotic compound to the area of pathology and site of action with the help of the reference ¹H images. To aid quantitative assessment of the local bioavailability of pharmacological, cosmetic and other fluorine compounds, external and internal standards containing different concentrations of ¹⁹F-containing compounds are used. As examples standards/phantoms containing the ¹⁹F-containing NSAID compounds 2-{[3-(Trifluoromethyl)phenyl]amino}benzoic acid (flufenamic acid) and 2-{[3-(trifluoromethyl)phenyl]amino}nicotinic acid (niflumic acid) can be used.

The invention concerns preferably the use of MR for a non-invasive localization and quantification of ¹⁹F-containing xenobiotic compounds in tissue up to several centimeters beneath the skin surface (thereby including muscles, joints and other deep seated tissue. As mentioned above MR analysis enables the detection of atomic nuclei and in our approach employs detection of MR signals derived from ¹⁹F nuclei in xenobiotic compounds containing one or more ¹⁹F atoms in their molecular structure. The invention therefore involves the application of ¹⁹F/¹H MRI to localize and quantify ¹⁹F-containing xenobiotic compounds, in particular those that are topically administered to the human body in the form of creams, ointments, lotions, drops etc. In contrast to the currently available technologies to measure the topical bioavailability of ¹⁹F-containing xenobiotic compounds, the ¹⁹F/¹H MRI approach will enable us to (i) quantify the local concentration/bioavailability of the pharmacological compound in body tissue irrelevant of depth and (ii) determine the proximity of the ¹⁹F-containing xenobiotic compound to the area of pathology and site of action with the help of reference ¹H MR images. This invention is focused on the determination of ¹⁹F-containing xenobiotic compounds. The transplantation of ¹⁹F-labeled cells as has been described previously [9] is distinct from this invention since it involves the transplantation of part of a donating organism into a host organism of the same species and preferably of the same immunological background.

Xenobiotics are foreign chemicals (eg. pharmaceutical substances) that are not normally produced or expected to be present in living organisms. Xenobiotic compounds may be referred to in the context of the present invention as "compounds". Essentially any compound administered to a subject comprising ¹⁹F could be considered as a xenobiotic compound, if the compound is not usually found endogenously in the region of the body used for examination. In contrast to the currently available technologies, this invention will enable us to image xenobiotic compounds in the human body at high submillimetric spatial resolution and high signal to noise ratio (SNR) even in areas several centimeters below the skin surface.

¹⁹F MRI is similar to ¹H MRI, both ¹⁹F and ¹H contain MR active nuclei with the only difference that ¹H nuclei (mostly in water but also in fat) are abundant in a living organism and fluorine is not present in a living organism in its carbon-bound form. This makes it possible to identify ¹⁹F-containing xenobiotics as background-free ¹⁹F images on ¹H anatomical scans. Basic MR experimentation is performed by placing a patient on the patient bed or a patient chair n front of the MR device, the coils are placed on the region of interest on the patient and both are moved towards the isocenter or any other position of the magnet. Measurements are started from a remote-controlled computer device and data analysis is identical to the clinical MR systems.

"Topical administration," "topically," and grammatical equivalents, refer to administration of a xenobiotic compound to a pre-defined or definite area of the body, such as to a defined or limited area of the skin surface, mucous membrane, a specified organ, a specified appendage or region (e.g., foot). Topical formulations include but are not limited to an ointment, a cream, a lotion, an oil, an emulsion, a gel, a paste, a milk, an aerosol, a powder, a foam, a wash, a transdermal patch and any combination thereof.

The invention also relates to measurement of the ¹⁹F-containing xenobiotic compounds after administration in the form of a composition.

A topical formulation or composition according to the invention may also comprise a dermatologically, cosmetic or pharmaceutically acceptable carrier, diluent or excipients in which the metal oxide coated active agent particulates may be e.g., dispersed or suspended. The coated active agent may be easily dispersed or suspended in such a carrier, diluent or excipients, by for example mixing to achieve an effective dispersion or suspension. If necessary, high shear forces may be applied to facilitate fast and efficient mixing of the coated particles in the carrier.

In other embodiments, the carrier of a composition of the invention is in the form of a gel, a cream, a lotion, a cleanser, a saturated pad, a plaster, a tape, an ointment, an oil, an emulsion, a paste, a milk, an aerosol, a powder, a foam, a wash, a dispenser etc. In other embodiments, the carrier of a composition of the invention is an oil-in-water cream. In still further embodiments, the dispersing phase is aqueous based and comprises water as a dispersing medium.

It should be noted that a composition of the invention may comprise a further at least one additive, such as but not limited to: a humectant (such as for example propylene glycol, glycerin, butylen glycol or polyethylene glycol), a buffer (such as for example citric acid aqueous solution, ammonium hydroxide solution phosphate buffer, borate buffer or carbonate buffer), a lubricant (such as for example cyclomethicone, dimethycone, castor oil, Iso propyl miristate, caprylic/capric triglyceride or octyl octanoate), an emulsifier (such as for example cetyl alcohol, glyceryl stearate, PEG-75 stearate, Ceteth-20, Steareth-20, Bis-PEG/PPG-16/16 PEG/PPG- 16/16 dimethicone, sorbitan mono-oleate or alkyl poly glucoside), a moisturize (such as for example sodium PCA, sodium hyaluronate, panthenol or sodium latate), a soothing agent (such as for example natural herbal extracts such as Anthemis Nobilis flower extract, Chamomilla Recutita, Hamamelis Virginiana, burdock root, Argireline, Arnica Montana Extract, Shea Butter or aloe vera), a perfume, an exfoliating agent (such as for example polyethylene, glycolic acid 70%), a filler, an anti-irritating agent (such as for example allantoin), a chelating agent (such as for example EDTA), a preservative (such as for example imidazolidinyl urea, potassium sorbate, phenoxyethanol, methyl paraben, propyl paraben or benzyl alcohol), a detergent (such as for example polysorbate 20, sodium dodecyl sulfate or ceterimonium chlorid), a coloring agent, an antimicrobial agent (such as for example SD alcohol 40 or Chlorhexidine gluconate), a thickening agent (such as for example xanthan gum, guar gum, carboxy methyl cellulose, Carbomer or ethyl cellulose) and any combinations thereof. In some embodiments, the formulations include a controlled- release device or composition where one or several of the components comprised in a composition of the invention are being released in a delayed fashion.

A composition of the invention may be formulated in a solid, semi-solid, or liquid form such as, e.g. suspensions, aerosols, or the like or any other formulation known to a person skilled in the art. In some embodiments, the compositions are administered in unit dosage forms suitable for single administration of precise dosage amounts. The compositions may also include, depending on the formulation desired, pharmaceutically-acceptable carriers as defined above. In some other embodiments of the present invention, a composition of the invention may be administered in a single dosage form comprising all the components together.

The term "administering" or its other lingual forms as used in the context of the present invention relates to the path by which an agent, a drug, fluid or other substance is brought into contact with the body.

In some embodiments of the present invention compositions may be provided as sustained release or timed release formulations.

Topical administration relates in a preferred embodiment to application of any given agent to the skin of a subject. Mammalian skin is made up of various layers, including the epidermis, the basement membrane, the dermis, the papillary region, reticular region and the hypodermis, which borders on muscle tissue. In general the epidermis is between 0.01 and 0.5 mm thick, the dermis between 1 and 5 mm thick and the hypodermis between 3 and 100 mm thick. The hypodermis contains a significant portion of the body's fat reserves and can represent and substantial thickness lying just below the dermal layer. The present invention is particularly advantageous in light of the high sub-millimetric resolution of the MRI analysis, which enables determination and quantification of the distribution of any given ¹⁹F-containing compound within the afore-mentioned layers of skin.

### FIGURES

The figures provided herein represent examples of particular embodiments of the invention and are not intended to limit the scope of the invention. The figures are to be considered as providing a further description of possible and potentially preferred embodiments that enhance the technical support of one or more non-limiting embodiments.

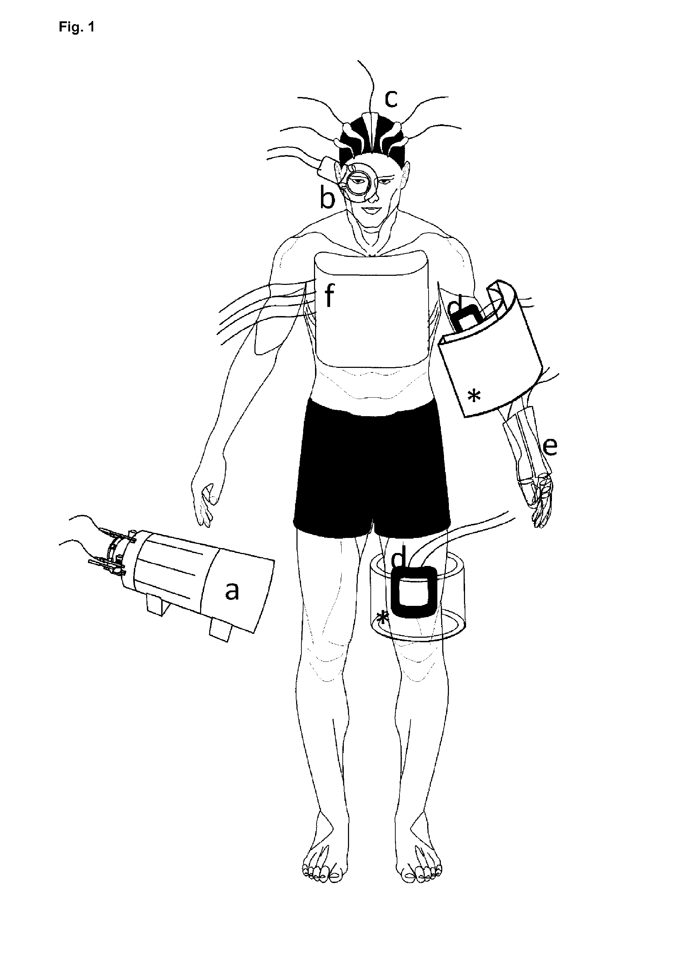

**Figure 1****:** Invention involving an array of RF coil setups (a-e) for use in conventional MR or portable (for example Halbach design) magnets (*) to perform topical drug MR bioavailability studies. Examples of coils include (a) birdcages for skin/joint application (b) loop surface coils for eye/nose application (c) head/scalp coils (d) skin patch coils with sustained-release drug formulations (e) glove design for finger/hand/wrist imaging (f) multichannel chest/heart/lung surface coils for inhaled agents but also inflatable, foldable and micro RF coils which can be used intravascular or introduced to any cavity of the body. The RF coil design is not limited to a rigid configuration but includes flexible designs using flexible wires or other MR safe and MR compatible conductors.

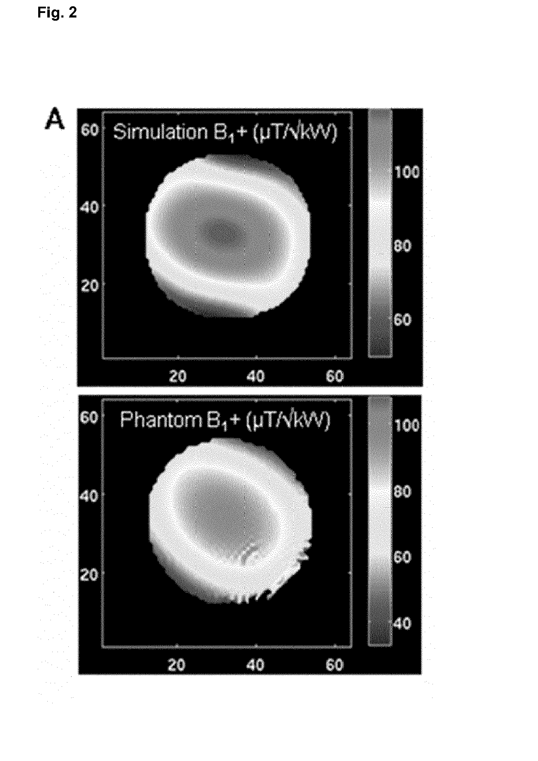

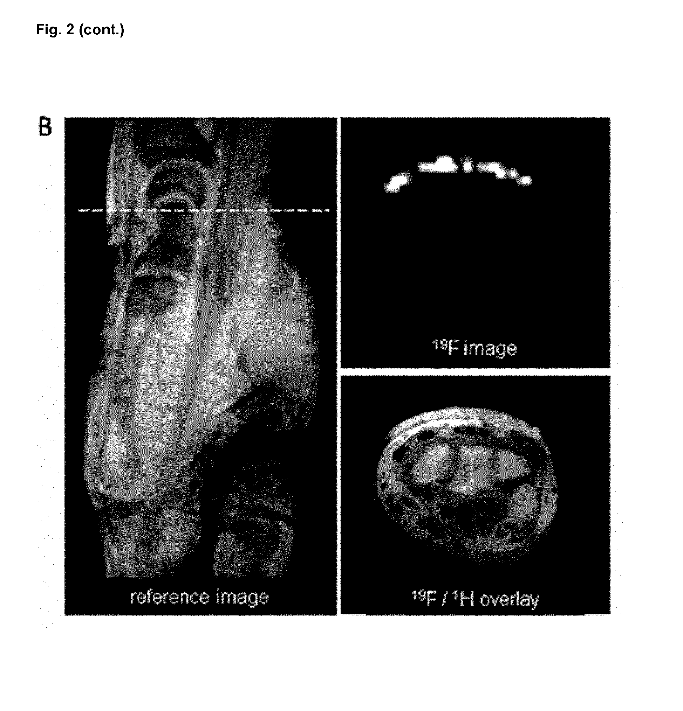

**Figure 2****:** Feasibility studies for employing a ¹⁹F/¹H birdcage resonator on a 7T human MR scanner to non-invasively measure topical drug bioavailability via in vivo ¹⁹F/¹H MR. (A) Simulated and measured/phantom B1 field measurements show a homogeneous distribution in the center of the ¹⁹F/¹H birdcage resonator. (B) In vivo ¹⁹F/¹H MRI showing a healthy volunteer's hand/wrist after topical application of a cream containing the NSAID flufenamic acid (2-{[3-(Trifluoromethyl)phenyl]amino}benzoic acid). Left image: sagittal reference IH MR image of the hand/wrist, dotted line depicts the position of the transverse MR images shown on the right; Upper right image: threshold filtered ¹⁹F GRE image; Lower right image: overlay of the ¹⁹F GRE image with the corresponding ¹H transversal slice of the wrist.

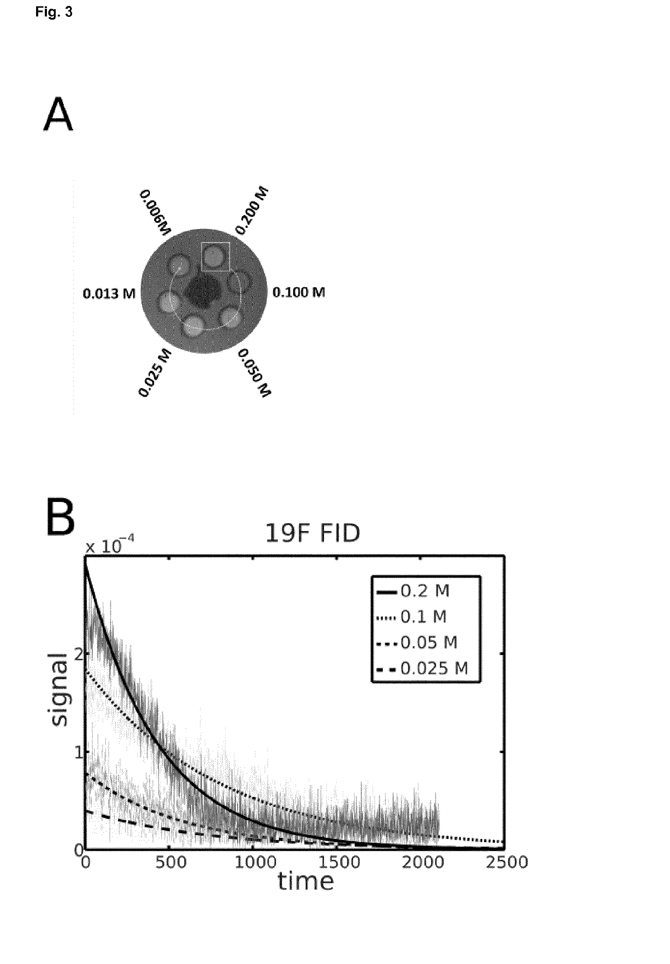

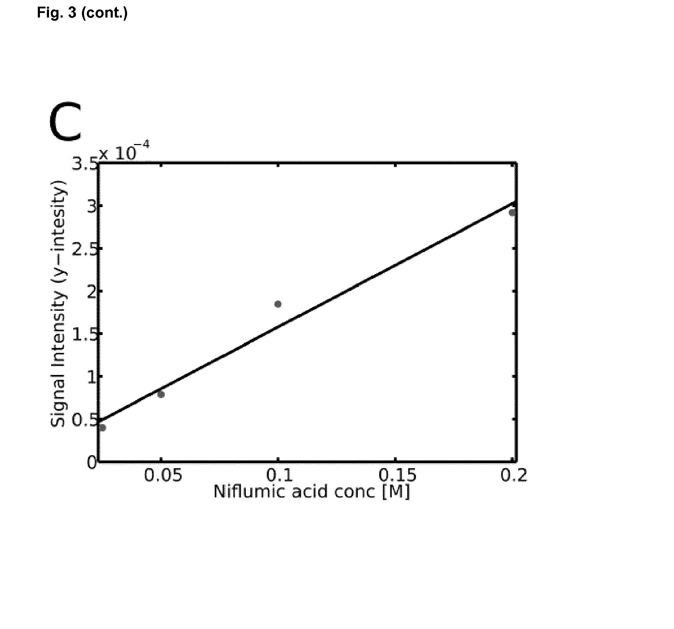

**Figure 3****:** ¹⁹F Single Voxel Spectroscopy for the quantification of the bioavailability of ¹⁹F-containing flufenamic acid (A) ¹H MR image showing the phantom construction and distribution of different concentrations of niflumic acid (0.006 - 0.200 M), the region of interest for SVS of the single concentrations is chosen (red box) for ¹⁹F spectral analysis (B) ¹⁹F signal intensity is measured as free induction decay (FID) in arbitrary units for each single voxel corresponding to the different niflumic acid concentrations (FOV of each voxel = 6.3x6.3x15mm, 128 averages, TRTTE=1500/30) (C) Signal intensity (calculated from the y-ntercept of the FID in B) versus concentration fit for the quantification of niflumic acid in vivo.

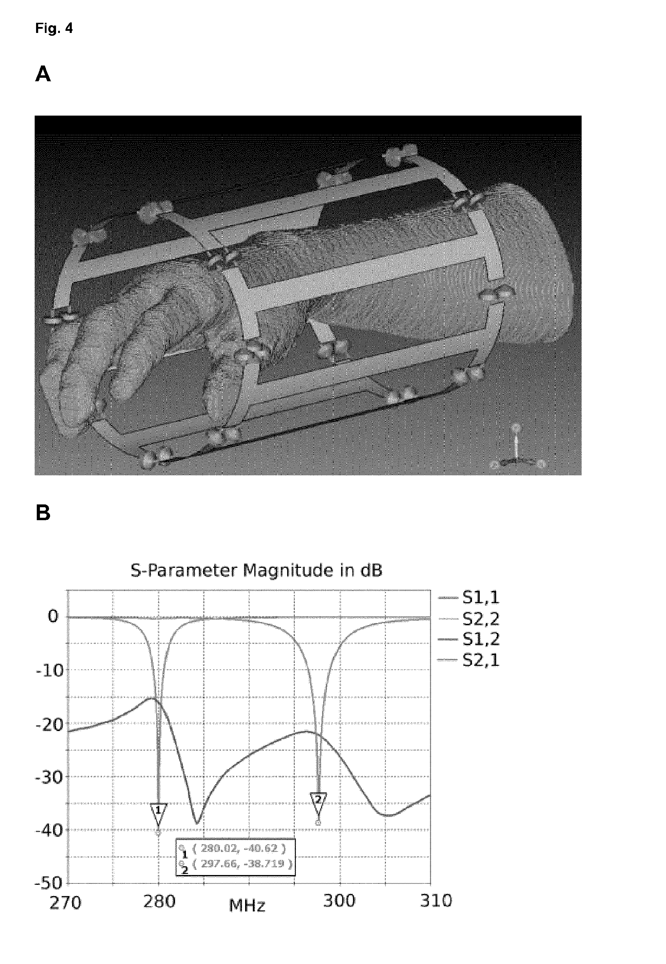

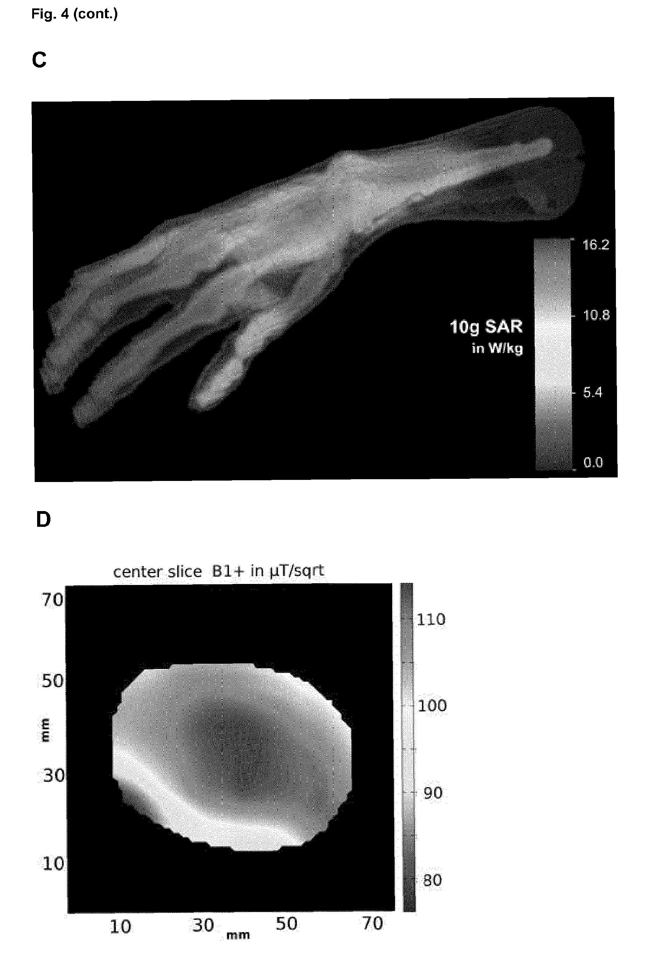

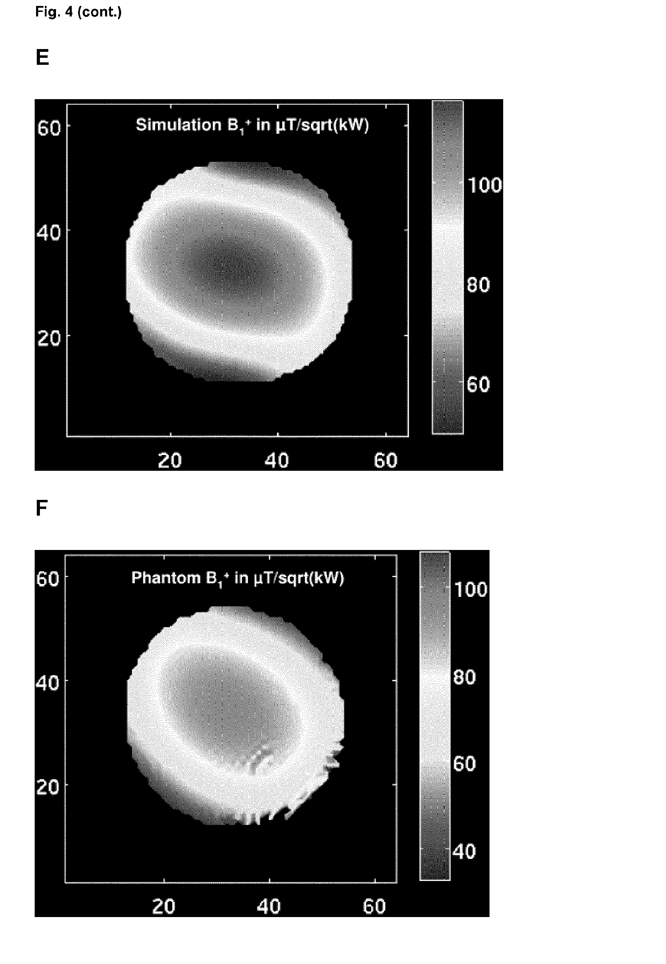

**Figure 4****:** A) model of the ¹⁹F/¹H birdgage with the right arm of the voxel model "Billie"; B) simulated S-Parameters of both ¹⁹F and ¹H-channel; C) 3D MOP projection of the 10g SAR (specific absorption rate) on the voxel model's arm; D) simulated B₁* field in the center of the coil using the voxel model; E) simulated and F) measured B₁* distribution in the center of the coil in a homogeneous phantom.

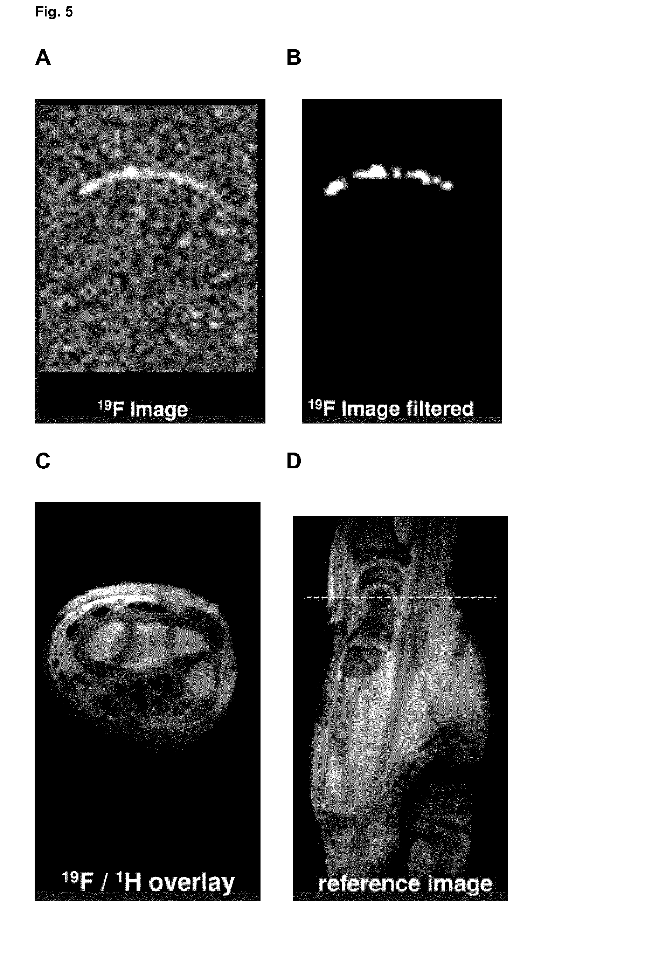

**Figure 5****:** A) original ¹⁹F GRE image; B) masked and threshold filtered ¹⁹F GRE image; C) overlay of the ¹⁹F GRE image with the corresponding transversal slice of the wrist; D) sagittal reference image, dotted line depicts the position of the transversal slice.

### EXAMPLES

The examples provided herein represent practical support for particular embodiments of the invention and are not intended to limit the scope of the invention. The examples are to be considered as providing a further description of possible and potentially preferred embodiments that demonstrate the relevant technical working of one or more non-limiting embodiments.

Rheumatoid arthritis (RA) is a chronic and systemic inflammatory condition of the skeletal system that also affects adults within the prime of their work productivity. By targeting synovial tissue, cartilage and bone, RA is a debilitating condition since it significantly hinders the physical functioning and working capacity of an individual [10]. While the therapeutic options available for treating RA is extensive, a comprehensive diagnosis of the disease (particularly knowledge of the exact location of inflammation) during early stages of disease is central for preventing and delaying further disease progression. New emerging MRI technologies to study cartilage composition including gadolinium enhanced cartilage imaging, ²³Na-MRI and T₂ relaxation mapping have been disclosed [11]. ¹⁹F-MRI has become increasingly important for small animal imaging in multiple fields of pre-clinical research including cell tracking and detection of inflammation [12]. Since ¹⁹F containing molecules are scarce in the human body, administration of exogenous fluorine containing compounds such as non-steroidal anti-inflammatory drugs (NSAIDs) will give a background free signal in ¹⁹F MRI. This example involves the development of a double-tuned ¹H/¹⁹F birdcage resonator and examines its applicability for hand and wrist ¹⁹F imaging at 7T following topical application of the NSAID 2-{[3-(Trifluoromethyl) phenyl]amino}benzoic acid.

Electromagnetic field (EMF) simulations with CST MWS (CST AG, Darmstadt, Germany) (Figure 4A) were performed using the right arm of the voxel model "Billie" of the Virtual Family [13]. Simulations were conducted to assess SAR (Figure 4C) and B₁* (Figure 4D) distribution. The coil was built using a design derived from initial simulations. Extended simulations were then performed for the final coil geometry. The 8-leg high-pass birdcage has a diameter of 10 cm and a length of 16 cm, one port was tuned to 279 MHz for ¹⁹F and the other one to 297 MHz for ¹H (Figure 4B). Phantom and in vivo measurements were performed on a 7 T Siemens Magnetom using a 3D gradient-echo sequence modified for ¹⁹F application. (GRE 3D TR/TE=15/1.0 ms, Matrix 48x48, FOV 100x100, Slab 80mm, 16 slices 5mm, 64 averages, TA 12:20min). Proton images were acquired using a T₁ weighted turbo spin-echo sequence (TSE TR/TE=400/9ms, Matrix 384x384, FOV 100x100, 16 slices 5mm, TA 1 min).

SAR calculations from the EMF Simulation show that the maximum 10g local SAR of 16.2W/kg (@4W stimulated power) lies well within IEC 60601-2-33 limits (Figure 4C). Simulated B₁* gives a maximum of 114µT/sqrt(kW) in the center of the voxel model (Figure 4D) and 116µT/sqrt(kW) in a phantom with a relative permittivity of e = 78 and conductivity of σ = 0.3S/m (Figure 4E). The measured B₁* on the proton channel is 97µT/sqrt(kW) in the center of a cylindrical phantom with similar properties as used in the EMF-Simulation (Figure 4F). Figure 5 shows first in vivo images acquired with the present dual-tunable ¹⁹F/¹H-birdcage at 7 T. Twenty minutes prior to imaging, the wrist of the volunteer received a 10g topical application of a cream containing the active compound 2-{[3-(Trifluoromethyl) phenyl]amino]benzoic acid at a concentration of 1mmol/l. Figure 5A shows the original ¹⁹F GRE image and Figure 5B the masked and threshold filtered image of the same slice. Figure 5C shows the overlay of a T₁W TSE proton image with the filtered ¹⁹F image. The dotted line in the reference image (Figure 5D) depicts the position of the transversal slice in Figure 5A-C.

The preliminary in vivo images acquired by the double-tuned ¹H/¹⁹F birdcage resonator demonstrate the feasibility of hand- and wrist-imaging at 7 T. The diagnostic quality of the acquired proton images is sufficient for analysis in patients with inflammatory rheumatoid disease of the hands and wrist. The initial ¹⁹F images of fluorine-containing NSAIDS such as 2-{[3-(Trifluoromethyl) phenyl]amino]benzoic acid are encouraging, and demonstrate that the present invention, applying ¹⁹F-MRI and NSAID therapy to the field of theranostics for visualizing and measuring the concentration of the therapeutically active compound reaching the inflammatory site in RA patients, provides a substantial improvement over previously known strategies.

Using a ¹⁹F/¹H birdcage resonator we have performed phantom and in vivo ¹⁹F/¹H MR measurements with a human MR scanner. Prior to in vivo ¹⁹F/¹H MR measurements (Figure 2B), we applied 10g of a cream containing flufenamic acid (1 mmo1/1 concentration) topically to a healthy wrist for 20 minutes. The overlaid in vivo ¹⁹F/¹H MR images (Figure 2B) show the feasibility of the current approach to localize ¹⁹F-containing xenobiotic compounds applied to the skin. By using multiple RF coils (e.g. coil arrays containing ¹⁹F and ¹H resonant coils) for transmission and reception, we are able to excite and receive interleaved or simultaneous proton ¹H anatomical images and ¹⁹F images. Furthermore, the transmission field (B1 field) can be adapted in a way that the target area will be homogeneously excited, to maximize the ¹⁹F signal yield from that area which will enable us also to acquire diagnostic ¹H MR-images.

One additional implementation of our approach utilising the concept of the invention involves ¹⁹F MR spectroscopy methods such as Single Voxel Spectroscopy (SVS) and Chemical Shift Imaging (CSI), which serve to quantify the 19F signal arising from the topically-applied ¹⁹F-containing xenobiotics in vivo. For this, phantoms with different concentrations of the ¹⁹F-containing compound eg. niflumic acid (Figure 3A) are prepared. SVS of each drug concentration (Figure 3B) is performed by placing a voxel around the region of interest and the ¹⁹F signal intensity measured from the free induction decay (FID) (Figure 3B). Each FID corresponds to a different concentration of niflumic acid. The signal intensity (calculated from the y-intercept of the FID) and the niflumic acid concentrations are plotted against each other to generate a concentration curve (Figure 3C). With this we can quantify the amount of ¹⁹F-containing compound eg. niflumic acid in vivo.

Additionally, further experimentation shows that the preferred embodiments of the invention provide surprising and unexpected effects, thereby solving the problem of the invention in a non-obvious fashion. For example early prototypes of inflatable, foldable or micro RF coils which can be used intravascular or introduced to any cavity of the body were tested. Also the RF coil design was moved from a rigid configuration to flexible designs using flexible wires or other MR safe and MR compatible conductors.

### References

1. Rougier A, Dupuis D, Lotte C, Roguet R, Schaefer H (1983) In vivo correlation between stratum corneum reservoir function and percutaneous absorption. J Invest Dermatol 81: 275-278.
2. Au WL, Skinner M, Kanfer 1 (2010) Comparison of tape stripping with the human skin blanching assay for the bioequivalence assessment of topical clobetasol propionate formulations. J Pharm Pharm Sci 13: 11-20.
3. Schmidt S, Banks R, Kumar V, Rand KH, Derendorf H (2008) Clinical microdialysis in skin and soft tissues: an update. J Clin Pharmacol 48: 351-364.
4. Korinth G, Jakasa 1, Wellner T, Kezic S, Kruse J et al. (2007) Percutaneous absorption and metabolism of 2-butoxyethanol in human volunteers: a microdialysis study. Toxicol Lett 170: 97-103.
5. Wascotte V, Caspers P, de Sterke J, Jadoul M, Guy RH et al. (2007) Assessment of the "skin reservoir" of urea by confocal Raman microspectroscopy and reverse iontophoresis in vivo. Pharm Res 24: 1897-1901.
6. Medendorp JP, Paudel KS, Lodder RA, Stinchcomb AL (2007) Near infrared spectrometry for the quantification of human dermal absorption of econazole nitrate and estradiol. Pharm Res 24: 186-193.
7. Yano T, Nakagawa A, Tsuji M, Noda K (1986) Skin permeability of various non-steroidal anti-inflammatory drugs in man. Life Sci 39: 1043-1050.
8. Ruiz-Cabello, et al. NMR Biomed, 2011 24:114-129
9. Ahrens ET, Flores R, Xu H, Morel PA (2005) In vivo imaging platform for tracking immunotherapeutic cells. Nat Biotechnol 23: 983-987.
10. Burton W Monison A, Maclean R, Ruderman E (2006) Systematic review of studies of productivity loss due to rheumatoid arthritis. Occup Med (Lond) 56: 18-27.
11. Borrero CG Mountz JM, Mountz JD (2011) Emerging MRI methods in rheumatoid arthritis. Nat Rev Rheumatol 7: 85-95.
12. Ruiz-Cabello J, Bameft BP, Bottomley PA, Bulte JW(2011) Fluorine (19F) MRS and MRI in biomedicine. NMR Biomed 24:114-129.
13. Christ A et al. (2010) The Virtual Family -development of surface based anatomical models of two adults and two children for dosimetric simulations. Phys MedBio|.2010,55: N23-38

## Claims

1. Non-invasive method for localisation and/or quantification of ¹⁹F-containing xenobiotic compounds via ¹⁹F magnetic resonance (MR) analysis after topical administration of said compound to a subject.

2. Method according to claim 1, **characterised in that** the MR is ¹⁹F/¹H MRI.

3. Method according to any one of the preceding claims, whereby proton ¹H anatomical images and ¹⁹F images are combined and/or compared, for example by overlay.

4. Method according to any one of the preceding claims, whereby the MR analysis is T1-, T2- and/or diffusion-weighted MR.

5. Method according to any one of the preceding claims, whereby the MR analysis is carried out with an MR device of 7 T or above, preferably 7 T or 14 T.

6. Method according to any one of the preceding claims, whereby the ¹⁹F-containing xenobiotic compound is Glucocorticosteroid (GC) or Non-Steroidal Anti-Inflammatory Drug (NSAID).

7. Method according to any one of the preceding claims, whereby the subject is an animal, preferably a mammal, more preferably a human.

8. Method according to any one of the preceding claims, whereby the local concentration and/or bioavailability of a pharmacological compound in body tissue is determined irrelevant of depth from the administration site of said compound.

9. Method according to any one of the preceding claims, whereby the location of disease- or pathologically-related physiological features, such as areas of inflammation, infection, or other pathologically relevant physiological feature, is additionally determined using ¹H MR analysis.

10. Use of the method according to any one of the preceding claims for the determination of bioavailability of a ¹⁹F-containing xenobiotic compound after topical administration of said compound to a subject.

11. Use of the method according to any one of the preceding claims for optimising the bioavailability of a topically applied ¹⁹F-containing xenobiotic compound by comparing the in vivo distribution of said compound after administration in multiple pharmaceutically acceptable formulations followed by subsequent modification of said formulations.

12. System for a localisation and/or quantification of ¹⁹F-containing compounds comprising single (¹⁹F) or multi-tunable (for example ¹⁹F/¹H, ¹⁹F/¹H/²³Na, ¹⁹F/¹H/³¹P) RF coils and an MR apparatus.

13. System according to claim 12, whereby according to the anatomy of the region of interest different coils are applied to detect and acquire the different signals and to quantify the bioavailability of ¹⁹F-containing compounds.

14. System according to claim 12 or 13, whereby the system comprises a double-tuned ¹⁹F/¹H birdcage resonator designed for hand and wrist anatomy.
